# EUROPEAN PATENT SPECIFICATION

(11) **EP 3 344 359 B1**
(45) Date of publication and mention of the grant of the patent: **27.11.2019**
(21) Application number: 16762848.6
(22) Date of filing: 01.09.2016
(51) Int. Cl.: B01D 33/01

(54) **FILTRATION DEVICE**
FILTRIERUNGSVORRICHTUNG
DISPOSITIF DE FILTRATION

(30) Priority: 02.09.2015 GB 201515531
(43) Date of publication of application: 11.07.2018
(73) Proprietor: Ngachi Limited, Birmingham, West Midlands B3 3HT (GB)
(72) Inventor: YIP, Nga Chi Lydia, Birmingham West Midlands B17 0ES (GB)
(74) Representative: HGF Limited
(86) International application number: PCT/GB2016/052706
(87) International publication number: WO 2017/037461

(56) References cited:
- GB-A- 2 329 852
- US-A1- 2005 123 895
- US-A1- 2011 168 644
- US-A1- 2014 110 356

## Description

This invention relates generally to a filtration device. More specifically, although not exclusively, this invention relates to a biological sample filtration device and method for filtering biological particulates from a suspension liquid.

The separation of particulates from a liquid sample is well-known, where the liquid sample may be, for example a solution, a suspension, a colloid, a dispersion or the like. There may be a requirement to perform this separation in a laboratory or in a field setting. Some separation methods involve the use of centrifuges, whereby particulate matter is separated from a liquid sample by centrifugal forces. Another method of separation is filtration, whereby a liquid sample is passed through a filter membrane, which may be formed of multiple layers each having openings of a predetermined size. Particulate matter which is greater in size than the size of the openings does not pass through the filter membrane and is therefore isolated from the filtrate.

Filtration is known to be used for the separation of biological, biochemical and environmental samples, amongst others, into constituent filtrates and particulates. For the avoidance of doubt, the term "biological" in relation to biological samples and particulates is intended to encompass, inter alia, biochemical samples and particulates and the like.

US2014/110356 describes a filtration device, GB2329852 describes a portable water filter with movable filter element, and US2011/168644 describes systems and methods for personal water filtration.

GB2392854 describes a filtration apparatus in which a filter membrane is pushed through a liquid sample which is held in a container. The filter membrane is sealed against the inner surfaces of the container so that passage of the filter through the liquid sample defines a boundary between the original liquid sample and the generated liquid filtrate. The container has an open top, facilitating access to the liquid filtrate. An unfiltered portion of the liquid sample will always remain at the base of the container, where the filtered particulate matter will remain immersed in liquid sample. Whilst useful in many circumstances, the applicants believe that the apparatus described in this document could be improved.

It is a first non-exclusive object of the invention to provide an improved filtration device for filtering biological samples which alleviates or mitigates one or more of the problems associated with prior art designs. It is a further, more general non-exclusive object of the invention to provide a filtration device for filtering supernatant in which the filtered particles are retained separate from the fluid of the filter sample.

Accordingly, a first aspect of the invention provides a filtration device as described in claim 1.

The use of a lid that closes the container prevents contaminants from entering into the device. The use of a sealed lid further enables the device to be inverted in use, thereby enabling the use of gravity to further assist the filtration and isolation of biological particulates.

The invention provides a filtration device, for example a biological sample filtration device, comprising a container with an open end and a closed end which is integral with the container, a lid for closing, e.g. sealingly closing, the open end of the container, a piston reciprocable within the container between the open end and the closed end and an actuating element connected to the piston and extending through a hole in the lid, the piston comprising a filter element bound by a peripheral portion with sealing means for sealingly engaging the container such that movement, in use, of the piston within the container forces a suspension fluid through the filter element but prevents or at least inhibits the passage of biological particulates, e.g. of a predetermined size or size range, therethrough, wherein the device comprises a further sealing means between the lid and the actuating element, thereby to enable the device to be oriented with the lid of the container lowermost without allowing egress of suspension fluid.

The device may comprise a sealing means, e.g. a second sealing means, between the lid and the container.

At least a portion of the lid may comprise a resilient and/or flexible and/or elastomeric material, e.g. for sealingly engaging with the open end of the container and/or for sealingly engaging with the actuating element. In some embodiments, substantially the entire lid is formed of a resilient and/or flexible and/or elastomeric material, e.g. for sealingly engaging with each of the open end of the container and the actuating element. In other embodiments, the lid comprises a portion, e.g. a first portion, formed of a resilient and/or flexible and/or elastomeric material, e.g. for sealingly engaging with the open end of the container. Additionally or alternatively, the lid may comprise a portion, e.g. a second portion, formed of a resilient and/or flexible and/or elastomeric resilient material, e.g. for sealingly engaging with the actuating element. Additionally or alternatively, the lid may comprise one or more portions or further portions formed of a different, e.g. more rigid and/or less flexible or less resilient material, which portions may be formed with or secured to one or both of the resilient and/or flexible and/or elastomeric portions, e.g. to form a unitary structure.

The container may comprise a formation, e.g. about at least a portion of the open end, for engaging with the lid, for example a cooperating formation in or on the lid. The formation of the container may comprises an inwardly or outwardly projecting radial formation or projection or bead. The lid may comprise a top wall and a skirt depending therefrom, which skirt may comprise a corresponding formation, e.g. a recess or lip, for engaging the formation of the container.

Where the formation of the container comprises an inwardly projecting radial formation or projection or bead, the skirt may be configured and/or sized and/or dimensioned to be received within the open end of the container, for example in the form of a plug. Preferably, however, the formation of the container comprises an outwardly projecting radial formation or projection or bead, for example wherein the skirt is configured and/or sized and/or dimensioned to receive or fit over the open end of the container.

The lid may comprise a formation, for example another formation, at or adjacent to and/or extending inwardly of the hole, e.g. for engaging with the actuating element. The formation or further formation may comprise a seal, wiper element or wiper seal, which may comprise a radial seal, radial wiper element or radial wiper seal. The formation may be thin and/or taper to an edge, e.g. a sharp edge. The lid may comprise a valve means, for example through which filtrate may be introduced and/or removed. The valve means may comprise a valve, e.g. a one-way valve. The valve means may comprise a self-sealing and/or penetratable and/or pierceable portion or element. The valve means may comprise an aperture with a cooperating closure (e.g. a bung or cap).

The filter element may comprise a depth filter, e.g. a column filter, which may comprise one or more layers of filter membrane. One or more of the layers may have a different mesh size than one or more of the other layers, or all layers may have the same mesh size. One or more layers may be configured to selectively bind selected biological particulates by antibody, charge or substrate binding means. One or more layers of the filter membrane may be configured to bind, retain and/or capture one or more biological particles or classes thereof, which may be charged, e.g. electrically charged, for example negatively charged or positively charged. At least part of the filter element may be hydrophobic and/or at least part of the filter element may be hydrophilic. One or more layers of the filter membrane and/or the filter element may comprise hydrophobic material and/or hydrophilic material, for example which may be configured to, respectively, repel or attract water or water based solutions. At least part of the filter element, e.g. one or more layers of the filter membrane, may be configured to selectively retain, prevent passage of or allow passage of fluid or particulates having a pH value above or below a specific value (e.g. specific acidity or alkalinity) or between minimum and maximum specific values (e.g. specific acidity or alkalinity values). At least part of the filter element, e.g. one or more layers of the filter membrane, may be magnetic or magnetically charged or comprise one or more magnetic characteristics or materials, e.g. configured to selectively bind, retain and/or capture one or more magnetic or magnetisable particles.

The filter element may be configured to prevent passage therethrough of particulates, for example cells, mammalian, yeast, bacteria, protein, DNA and/or small molecules. The filter element may be configured to prevent the passage therethrough of particulates, for example biological particulates (e.g. larger than 1000 µm), eukaryotic cells (e.g. from 10-100 µm), bacteria (e.g. from 0.5-5 µm), viruses (e.g. from 250-400 nm), proteins (e.g. from 5-10 nm), lipids (e.g. from 1-5 nm) or small molecules (e.g. from 0.5-1 nm). Additionally or alternatively, the filter element may be configured to prevent the passage therethrough of particulates that are larger than 0.5 nm, for example larger than 1 nm or 5 nm, e.g. larger than 0.1 µm or 0.2 µm, such as larger than 9 µm or 100 µm or even larger than 1000 µm.

The filter element may comprise a mesh size of between about 0.5 nm and about 1000 µm, or may be greater than 1000 µm. The filter element may comprise a mesh size of between about 0.1 µm and about 100 µm, for example between about 0.1 µm and about 10 µm. The filter element may comprise a mesh size of, for example, about 0.1 µm or about 0.2 µm or about 9 µm or about 1000 µm. The filter element may comprise a mesh size of up to about 0.035 µm, e.g. between about 0.015 µm and about 0.03 µm, for example for filtration of viruses. The filter element may comprise a mesh size of between about 0.03 µm and about 0.1 µm, e.g. between about 0.035 µm and about 0.1 µm, for example for the filtration of bacteria.

Other useful filtration ranges will be appreciated by those skilled in the art. The filter element may comprise a mesh size of between about 0.15 µm and about 0.45 µm. The filter element may comprise a mesh size of between about 0.46 µm and about 1.6 µm. The filter element may comprise a mesh size of between about 1.65 µm and about 5 µm. The filter element may comprise a mesh size of between about 5.05 µm and about 10 µm. The filter element may comprise a mesh size of between about 10.05 µm and about 15 µm. The filter element may comprise a mesh size of between about 15.05 µm and about 20 µm. The filter element may comprise a mesh size of between about 20.05 µm and about 40 µm. The filter element may comprise a mesh size of between about 40.05 µm and about 100 µm. The filter element may comprise a mesh size of between about 100 µm and about 500 µm. The filter element may comprise a mesh size of between about 500 µm and about 1000 µm.

Additionally or alternatively, the filter element may comprise a solid phase extraction material, for example a c18 RP silica (e.g. for filtering small molecules) or an unmodified silica (e.g. for filtering lipids) or neutral or cation exchange (e.g. for filtering proteins).

The device may further comprise a stop, which may be adjacent the closed end. The piston may be configured to abut, e.g. in use, the stop, for example to provide a predetermined volume between the piston and the closed end. The closed end may taper, for example to provide a predetermined volume into which movement of the piston is prevented. The taper may comprise a straight or rounded taper, for example wherein the closed end is substantially conical or frustoconical or ellipsoidal or spherical or even pyramidal. In embodiments, the container comprises a centrifuge tube or a test tube.

The piston may comprise a hub, for example from which one or more spokes may extend. The one or more spokes may comprise two or more, e.g. three or more, spokes and/or may connect the hub to the peripheral portion. The actuating element may comprise a piston rod that may be connected at one of its ends to the hub and/or may include a handle or knob at its other end, e.g. for the application of a plunging force. The hub may be located at or adjacent one side of the peripheral portion, but is preferably located at the centre of the piston. In some embodiments, two or more or all of the peripheral portion, the spoke or spokes and/or the hub are integral and/or comprise a unitary structure, for example they may comprise an integral moulding.

At least part of the peripheral portion may comprise or be formed of a resilient and/or flexible and/or elastomeric material and/or may be larger than the inside of the container, for example thereby to provide the sealing means. In embodiments, the sealing means of the peripheral portion comprises one or more radial projections formed of a resilient and/or flexible and/or elastomeric material. In embodiments, the sealing means of the peripheral portion comprises an O-ring seal in a radial groove of the peripheral portion.

The peripheral portion may comprise one or more radial projections that may be spaced from the sealing means, e.g. along the thickness of the piston, thereby to locate and/or align and/or inhibit skewing of the piston. In embodiments, the one or more radial projections contribute to or form part of the sealing means. For example, the sealing means may comprise two or more radial projections spaced along the thickness of the piston.

One or more of the radial projections may comprise a resilient and/or flexible and/or elastomeric material.

Also disclosed, in examples, is that the closed end may comprise a lid or stopper configured to removably and/or sealingly close the closed end of the container. The lid or stopper may comprise one or more seals and/or a thread, e.g. a helical thread. Where the lid or stopper comprises a helical thread the container may be provided with a corresponding thread, e.g. a helical thread, configured to engage with the thread of the lid or stopper. Where the lid or stopper comprises one or more seals the seal or seals may be configured to engage with one or more surfaces of the container in a sealing manner.

Also disclosed herein is a method of filtering a biological sample, the method comprising urging a plunger through a hole in a lid of a container, e.g. a sealed container, to force a filter element bound by a peripheral portion, e.g. a peripheral sealing portion, along a container such that a suspension fluid is urged through the filter element whilst the passage of biological particulates, e.g. of a predetermined size or size range, therethrough is prevented or at least inhibited.

The plunger may comprise a piston, for example comprising the filter and the peripheral portion. The plunger may additionally or alternatively comprise an actuating element, which may extend through the hole in the lid.

Yet another aspect of the invention provides a method of filtering a biological sample, the method comprising inverting a sealed container having a lid sealed with respect to the container and with respect to an actuating element to prevent egress of suspension fluid and urging the actuating element through a hole in the sealed container to force a piston comprising a filter element bound by a peripheral sealing portion along a container such that a suspension fluid is urged through the filter element whilst the passage of biological particulates therethrough is prevented.

The container may contain, e.g. the method may comprise introducing or placing or adding into the container, a liquid sample and (optionally) air, e.g. between the piston and a closed end of the container. Preferably the liquid sample and air are in the container and/or between the piston and closed end before the container is inverted. Inverting the container may comprise orienting the container such that the liquid sample rests on the piston, e.g. with the air located between the liquid sample and the closed end of the container.

The method may comprise urging the actuating element such that the air is compressed to create a positive pressure that forces liquid from the liquid sample through the filter element.

The piston may be urged along the container to a stop or against a taper at or adjacent a closed end of the container to provide a predetermined volume containing filtered biological particulates. The method may comprise removing and/or adding filtrate or another fluid or material through a valve, e.g. a one-way valve, of, e.g. in or on, the lid.

The method may comprise removing and/or adding filtrate or another fluid or material through an aperture of an aperture and cooperating closure (e.g. a bung or cap) of, e.g. in or on, the lid. The method may comprise removing the closure, e.g. opening the aperture, at least partially from the aperture prior to removing and/or adding filtrate or another fluid or material through the aperture. The method may comprise replacing or installing the closure on or in or over the aperture subsequent to removing and/or adding filtrate or another fluid or material through the aperture.

The method may comprise removing and/or adding filtrate or another fluid or material through a self-sealing penetratable and/or pierceable portion or element of, e.g. in or on, the lid. The method may comprise piercing and/or penetrating the self-sealing penetratable and/or pierceable portion or element, e.g. with a piercing means (for example a syringe or needle). The method may comprise removing and/or adding filtrate or another fluid or material from beneath the lid or to underneath the lid (e.g. from or to one side of the lid to the other), for example using the piercing means and/or a further device. The method may comprise removing or retracting the piercing means from the self-sealing penetratable and/or pierceable portion or element, for example such that the self-sealing penetratable and/or pierceable portion or element seals closed (e.g. in a fluid tight manner).

The method may further comprise the suspension fluid being urged through the filter element such that biological particulates are retained within, e.g. captured or blocked within, e.g. adjacent or against one or more layers of, a depth filter, e.g. a column filter, of the filter element.

The method may comprise filtering one or more of large particulates (e.g. larger than 1000µm), biological particulates such as eukaryotic cells (e.g. from 10-100µm), bacteria (e.g. from 0.5-5µm), viruses (e.g. from 250-400nm), proteins (e.g. from 5-10nm), lipids (e.g. from 1-5nm) or small molecules (e.g. from 0.5-1 nm). Additionally or alternatively, the method may comprise filtering biological particulates are larger than 0.5 nm, for example between 0.5nm and 1 nm or 5nm or 10nm or 250nm or 400nm or 0.5µm or 5µm or 10µm or 100µm or 1000µm. The method may comprise filtering biological particulates are larger than 1nm, for example between 1 nm and 5nm or 10nm or 250nm or 400nm or 0.5µm or 5µm or 10µm or 100µm or 1000µm. The method may comprise filtering biological particulates are larger than 5nm, for example between 5nm and 10nm or 250nm or 400nm or 0.5µm or 5µm or 10µm or 100µm or 1000µm. The method may comprise filtering biological particulates are larger than 10nm, for example between 10nm and 250nm or 400nm or 0.5µm or 5µm or 10µm or 100µm or 1000µm. The method may comprise filtering biological particulates are larger than 250nm, for example between 250nm and 400nm or 0.5µm or 5µm or 10µm or 100µm or 1000µm. The method may comprise filtering biological particulates are larger than 400nm, for example between 400nm and 0.5µm or 5µm or 10µm or 100µm or 1000µm. The method may comprise filtering biological particulates are larger than 0.5µm, for example between 0.5µm and 5µm or 10µm or 100µm or 1000µm. The method may comprise filtering biological particulates are larger than 5µm, for example between 5µm and 10µm or 100µm or 1000µm. The method may comprise filtering biological particulates are larger than 10µm, for example between 10µm and 100µm or 1000µm. The method may comprise filtering biological particulates are larger than 100µm, for example between 100µm and 1000µm. The method may comprise filtering biological particulates are larger than 1000µm.

For the avoidance of doubt, any of the features described herein apply equally to any aspect of the invention. For example, the device may comprise any one or more features relevant to the method and/or the method may comprise any one or more features or steps relevant to one or more features of the device.

Also disclosed herein is a computer program element comprising and/or describing and/or defining a three-dimensional design for use with a three-dimensional printing means or printer or additive manufacturing means or device, the three-dimensional design comprising one or more components of an embodiment of the device described above.

The aforementioned computer program element may be embodied on a computer readable medium.

Further disclosed herein is a computer readable medium having the aforementioned computer program element stored thereon.

Within the scope of this application it is expressly intended that the various aspects, embodiments, examples and alternatives set out in the preceding paragraphs, in the claims and/or in the following description and drawings, and in particular the individual features thereof, may be taken independently or in any combination. That is, all embodiments and/or features of any embodiment can be combined in any way and/or combination, unless such features are incompatible. For the avoidance of doubt, the terms "may", "and/or", "e.g.", "for example" and any similar term as used herein should be interpreted as non-limiting such that any feature so-described need not be present.

Embodiments of the invention will now be described by way of example only with reference to the accompanying drawings in which:
Figure 1 is a perspective view of a filtration device according to a first embodiment of the invention;
Figure 2 is a perspective view of the filtration device of Figure 1 according to an embodiment of the invention;
Figure 3 is a section view of a first alternative piston sealing arrangement;
Figure 4 is a section view of a second alternative piston sealing arrangement;
Figure 5 is a section view of a third alternative piston sealing arrangement;
Figure 6 is a section view of an alternative embodiment of the filtration device;
Figure 7 is a section view of the filtration device of Figure 6 in an inverted orientation; and
Figure 8 is a section view of the filtration device of Figure 6 during further processing.

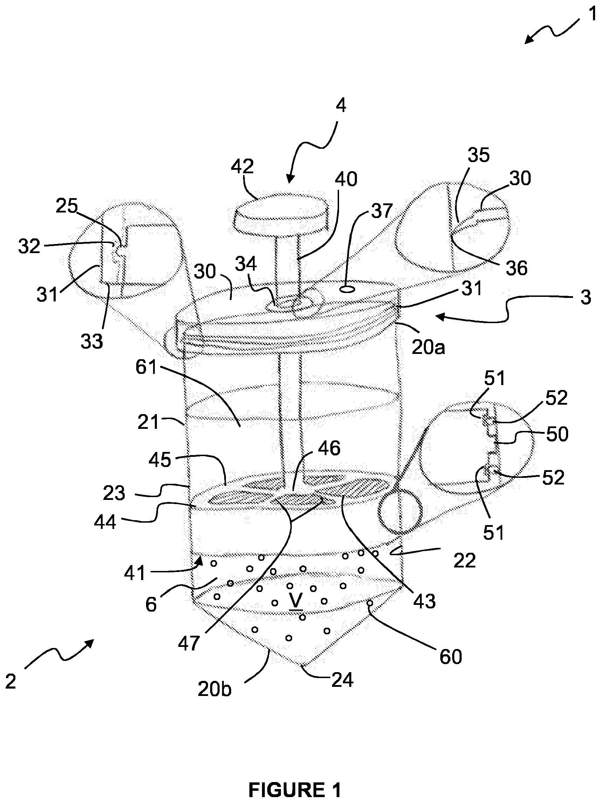

Referring now to Figure 1, there is shown a filtration device 1 according to one embodiment of the invention, including a container 2, a closure 3 and a plunger 4.

The container 2 has an open end 20a and a closed end 20b with a hollow cylindrical main body 21 connecting the two ends 20a, 20b. The main body 21 has a constant internal cross-sectional diameter, having an inner surface 22 and an outer surface 23. The closed end 20b of the container 2 has a frusto-conical shape in this embodiment, with the body 21 tapering towards a point 24. A known volume V is defined within the tapered closed end 20b.

The open end 20a of the container includes a continuous radial bead 25 projecting outwardly from the outer surface 23 of the open end 20a. In this embodiment, the container 2 is a centrifuge tube formed from borosilicate glass and with a generally circular cross-section. However, in alternative embodiments, the container 2 may comprise a test tube, a bespoke container or any other suitable receptacle and/or may have any suitable cross-sectional shape and/or may be formed from any suitable material, such as a plastics material having suitable properties.

The closure 3 is moulded from a single piece of rubber in this embodiment and includes a substantially flat cover 30 that is round in plan. A peripheral wall 31 depends from the outer edge of the cover 30. The peripheral wall 31 includes a recess 32 on its inner surface, configured to engage with the radial bead 25 of the container 2.

In use, the peripheral wall 31 of the closure 3 is pressed onto the open end 20a of the container 2, whereby a leading edge 33 of the peripheral wall 31 resiliently deforms over the radial bead 25 of the container 2 such that the recess 32 of the peripheral wall 31 resiliently captivates the bead 25 of the container 2. In this way the bead 25 is removably retained within the recess 32 of the peripheral wall 31. The recess 32 of the peripheral wall 31 is configured to engage in a circumferentially continuous and fluidly sealing fit against the radial bead 25 of the container 2.

The cover 30 includes an aperture 34 through its thickness, located centrally, with a wiper seal 35 projecting radially inwardly from the cover 30. The thickness of the wiper seal 35 tapers from the periphery of the aperture 34 to a sharp edge 36 at the innermost diameter of the seal 35. The closure 3 also includes a one-way evacuation valve 37 in the cover 30.

The plunger 4 includes an elongate actuating rod 40 connected at one of its ends to a piston 41 and removably connected at its other end to a handle 42. The rod 40 is in the form of an elongate tube having a constant external cross-sectional diameter along its length and is formed of a plastics material. The external diameter of the rod 40 is sized and dimensioned to engage the sharp edge 36 of the wiper seal 35, which deforms thereagainst to provide a fluidly sealed engagement with the outer surface of the rod 40.

The handle 42 is connected to the rod 40 via a threaded blind hole (not shown) in the lower surface of the handle 42, into which a threaded end of the rod 40 is engaged. The handle 42 is moulded from a plastics material and has a circular shape in plan, but it may have any shape suitable to its operation by the single hand of a user (not shown) and may be formed from any suitable material.

The piston 41 includes a filter element 43 surrounded by and releasably secured to a filter holder 44. The filter holder 44 includes a peripheral rim 45, which is circular in plan and is connected to a central hub 46 by four reinforcing spokes 47. The filter holder 44, which has a substantially flat major surface, is integrally moulded from a plastics material and is attached to an end of the rod 40 at the centre of the central hub 46.

The peripheral rim 45 has an external diameter which is marginally less than the inner cross-sectional diameter of the container 2. The outer circumference of the peripheral rim 45 includes a central radial guide 50 and a pair of circumferential grooves 51 on either side of the guide 50, one adjacent each of its major surfaces, each of which receives an O-ring seal 52. The guide 50 projects radially from the circumferential surface of the peripheral rim 45 such that an outer circumferential surface thereof contacts the internal diameter of the container 2. The guide 50 has a substantially featureless outer circumferential surface for free sliding engagement with the inner surface 22 of the container 2. Each of the O-ring seals 52 is sized to sealingly engage the inner surface 22 of the container 2 in order to provide a circumferentially continuous fluid seal between the peripheral rim 45 of the piston 4 and the inner surface 22 of the container 2.

The filter 43 is secured to the opposite side of the filter holder 44 from the rod 40, with the spokes 47 and peripheral rim 45 of the holder 44 configured to expose a major portion of the filter 43. The filter 43 in this embodiment includes a filter sheet formed from cellulose acetate, is circular in plan and includes multiple layers (not shown) of filter membranes. It is envisaged, however, that the filter sheet may be formed from nylon mesh, glass microfiber mesh, polyethersulphone, polypropylene, polytetrafluoroethylene mesh, cellulose mesh, cellulose acetate mesh, carbon filtrates, PVDF, membrane filters, polycarbonate, GHP, metal based mesh, fused silica gel filters or any other suitable material. The filter membranes have a pore size suitable for filtration of the selected biological sample. For example, the filter membranes have a pore size of between about 0.5 nm and about 1000 µm, or may be greater than 1000 µm. Alternatively, the pore size may be between about 0.1 µm and about 100 µm, e.g. about 0.1 µm to about 10 µm.

In use, a liquid sample 6 that includes biological particulates 60 in a liquid is poured by a user (not shown) into the container 2. The piston 41 is then inserted into the open end 20a of the container 2. The handle 42 is removed from the end of the rod 40, which is then pushed through the aperture 34 of the closure 3, so that the wiper seal 35 of the closure 3 engages with the outer surface of the rod 40. The closure 3 is then fitted onto the open end 20a of the container 2, with the resilient peripheral wall 31 of the closure 3 retained in sealing engagement against the projecting bead 25 of the closure 2, as described above. The handle 42 is then reattached onto the free end of the rod 40, which extends through the aperture 34 of the closure 3. The filtration device 1 is then ready for use and the closure 3 prevents any contaminants from entering into the liquid sample 6.

In operation, a user pushes the handle 42 towards the container 2 such that the rod 40 is pushed through the aperture 34 of the closure 3. The piston 41, attached to the other end of the rod 40, moves toward the closed end 20b of the container 2. The piston 41 is thus pushed along the inside of the container 2, into the liquid sample 6 contained within the container 2. The guide 50 of the piston 41 ensures that the major plane of the filter surface remains oriented perpendicularly to the inner surface 22 of the main body 21 of the container 2. As the piston 41 is pushed into the liquid sample 6, particulate matter 60 greater in size than the pore size of the filter is retained against the leading surface of the filter 43, whilst liquid and smaller particles pass through the filter 43 to form filtrate 61. The O-rings 52, as described above, provide a continuous circumferential fluid seal between the piston 41 and the inner surface 22 of the container 2, therefore preventing the passage of liquid or filtrate 61 therethrough.

The piston 41 is pushed along the container 2 until it abuts the taper of the closed end 20b of the container 2, at which point the piston 41 is constrained from further movement towards the closed end 20b. Thus, the remaining sample 6 in a volume V defined by the tapered closed end 20b contains mainly biological particulates 60.

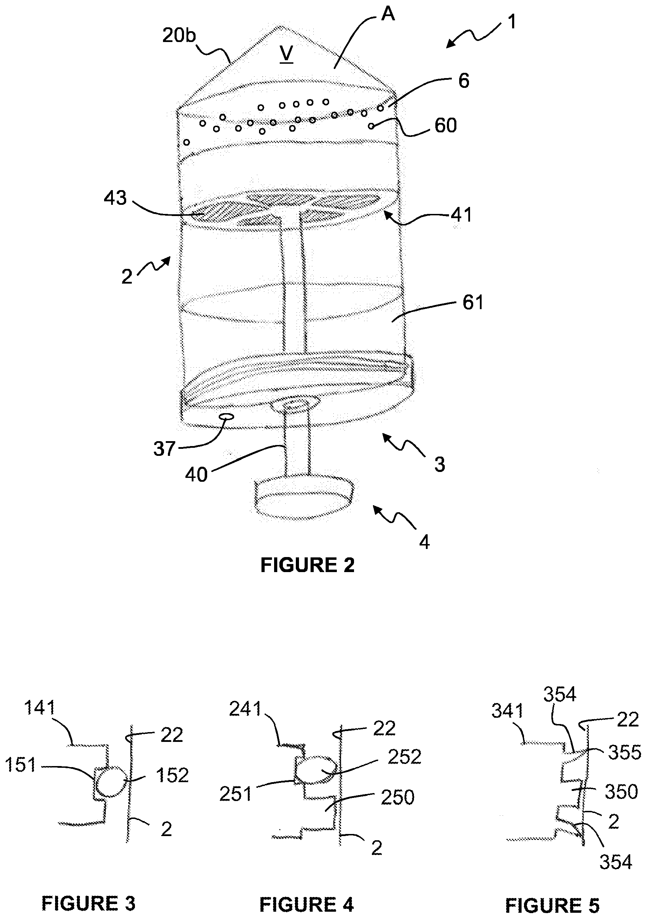

Referring now to Figure 2, a preferred method of use of the filtration device 1 is shown. In this embodiment, the filtration device 1 is prepared for use as described above but the piston 41 is positioned adjacent the closure 3 thereby to provide a volume of air between it and the liquid sample 6. The device 1 is then inverted in orientation, e.g. turned upside down, with the closure 3 lowermost, prior to actuation. The liquid sample 6 therefore rests on the piston 41 such that an air gap A is uppermost and the volume V is filled with air. The filtration device 1 is then operated in a similar manner to that described above.

Actuation of the piston 41 causes the liquid sample 6 to compress the air gap A, thereby creating a positive pressure that urges the liquid through the filter 43. In the inverted orientation of this embodiment of the invention the filtrate 61 does not remain in contact with the filter 43 but instead moves, due to gravity, towards the closure 3. As a consequence, there is no hydrostatic resistance inhibiting the passage of liquid filtrate 61 through the filter 43.

Thus, when the piston 41 abuts the taper of the closed end 20b, the volume V contains substantially only air A and particulate matter 60. The skilled person will appreciate that this arrangement is particularly advantageous for isolating the particulate matter 60 more effectively from the filtrate 61.

In this inverted orientation, the wiper seal 35 prevents egress of liquid sample 6 or filtrate 61 from between the rod 40 and the closure 3. Additionally, fluid is prevented from egress via the seal provided by the bead 25 of the container 2 and the recess 32 of the closure 3.

The filtrate 61 may then be removed from the filtration device 1 via the one-way valve 37 in the closure 3 using a suitable syringe (not shown). The closure 3 may then be removed from the open end 20a of the container 2 and any particulate matter which is retained on the leading surface of the filter 43 may be accessed. In this embodiment, the filter element 43 is removable, thereby enabling biological particulates remaining on the filter element 43 to be removed from the device 1 more easily.

It is further envisaged, in an alternative method of use, that the filtration device 1 is oriented as shown in Figure 1 and the piston 41 urged through a liquid sample 6 until the leading surface of the piston 41 abuts the taper of the closed end 20b of the container 2. The filtration device 1 is then inverted in orientation (e.g. turned upside down) as shown in Figure 2, whereby liquid from the sample 6 retained in the fixed volume V of the closed end 20b flows by the force of gravity through the filter 43.

Filtration using the method and orientation of the filtration device 1 shown in Figure 1 may require several subsequent stages of processing in order to fully isolate the filtered particulate matter from the remainder of the liquid sample or filtrate. Advantageously, the alternative methods of filtration (initial or subsequent inversion of the filtration device 1) mitigate these subsequent stages of processing and may remove the need for them entirely. Thus, filtering according to these methods may result in both time and cost savings.

### Examples

Referring now to Figure 3, there is shown an alternative sealing arrangement between a piston 141 and the inner surface 22 of the container 2. In this embodiment the piston 141 includes a single circumferential groove 151 into which is fitted an O-ring seal 152. The seal 152 according to this embodiment is similar in design to the seal 52 described above, but with a larger cross-sectional diameter. The dimensions of the seal 152 are selected in concert with the depth and configuration of the recess 151 so as to engage, in use, against the inner surface 22 of the container 2, thereby providing a circumferentially continuous fluid seal therebetween. Advantageously, this arrangement provides a simpler, more cost effective sealing means than that which is described above in relation to the device 1 of Figure 1.

Referring now to Figure 4, there is shown a further alternative sealing arrangement between a piston 241 and the inner surface 22 of the container 2. The piston 241 according to this embodiment includes a guide 250 and a single recess 251 on the upstream side of the guide 250. The guide 250 is similar to the guide 50 as described above in relation to the embodiment of Figure 1, but is offset to the downstream side of the piston 241. An O-ring seal 252 is fitted into the radial recess 251. When the piston 241 is moved within the container 2, the guide 250 ensures that the piston 241 maintains a generally perpendicular orientation with respect to the inner surface 22 of the container 2. The O-ring seal 252 is therefore maintained in engagement with the inner surface 22 of the container 2 around the entire circumference thereof.

Referring now to Figure 5, there is shown a further alternative sealing arrangement between a piston 341 and the inner surface 22 of the container 2. In this embodiment, the piston 341 includes a pair of radial wiper seals 354 and a radial guide 350. The wiper seals 354 are disposed on either side of the radial guide 350, and taper in cross-section from the piston 341 toward respective pointed edges 355. The pointed edges 355 are configured to engage with the inner surface 22 of the container 2 such that each seal 354 deforms thereagainst and provides a continuous circumferential fluid seal.

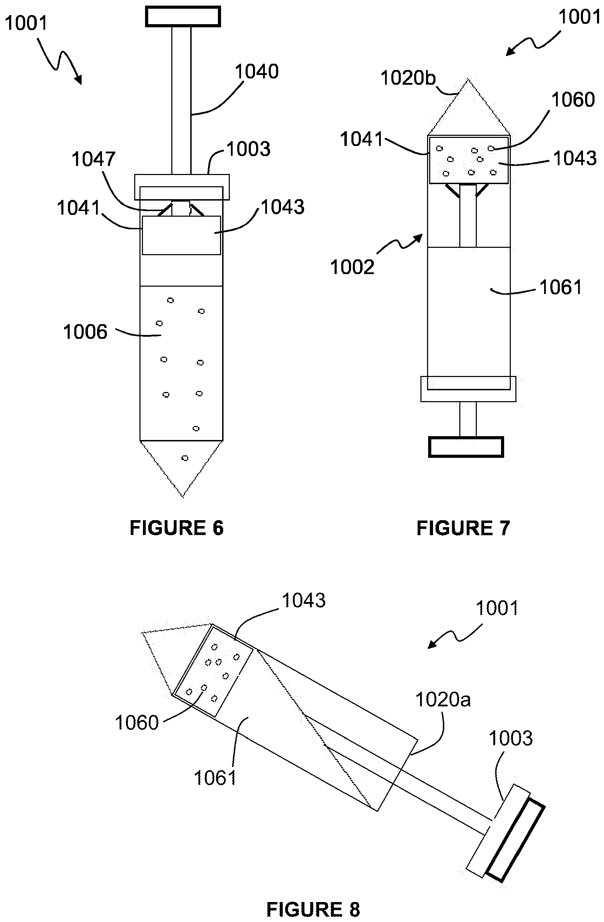

Referring now to Figure 6 there is shown a filtration device 1001 according to an alternative embodiment of the invention. The filtration device 1001 according to this embodiment differs from the filtration device 1 according to the first embodiment in that, inter alia, the piston 1041 includes a depth filter 1043 (having multiple layers of filter membranes) and reinforcing spokes 1047 project from the piston 1041 and are attached to the rod 1040 at locations above (in the orientation shown in Figure 6) the substantially planar face of the piston 1041. Advantageously, this configuration of spokes 1047 provides enhanced support between the piston 1041 and the rod 1040. The spokes 1047 may replace the spokes 47 described in relation to the embodiment of the invention shown in Figure 1 or, alternatively, the spokes 1047 may be provided in addition to the spokes 47.

In use, liquid sample 1006 is poured into the filter device 1001, which is then inverted as shown in Figure 7. The piston 1041 is then urged through the liquid sample 1006 until the piston 1041 abuts the closed end 1020b of the container 1002. Particulate matter 1060 is retained within the depth filter 1043. As shown in Figure 8, the closure 1003 is then removed from open end 1020a of the container 1002 and the filtrate 1061 is removed from the filtration device 1001.

It will be appreciated by those skilled in the art that several variations to the aforementioned embodiments are envisaged without departing from the scope of the invention. For example, the container 2 may include a radial projection from its inner surface 22 adjacent to its closed end 20b to provide a stop for the piston 41. The radial projection may be configured to engage against a leading surface of the piston 41 such that the piston 41 is restrained from movement beyond the projection. In such embodiments, the known volume V might be defined as the internal volume of the container 2 between the piston 41 or the radial projection and the closed end 20b. Additionally or alternatively, the closed end 20b of the container 2 may be flat and/or planar.

The closure 3 may comprise or be replaced by a plug portion or bung configured to fit sealingly within the open end of the container 2. In some embodiments, the plug portion or bung may comprise a cylindrical portion or an annular wall, for example depending from the cover 30 and/or may be formed from a resilient material such as rubber. The closure 3 need not be formed completely of a resilient material and/or the valve 37 may be omitted. Other variations are also envisaged, as would be appreciated by the skilled person.

Additionally or alternatively, the closure 3 may comprise an aperture and cooperating bung or cap. The cooperating bung or cap may be retained or retainable by the closure 3, for example by way of a tether extending between and configured to connect the bung or cap to the closure 3.

Additionally or alternatively, the closure 3 may comprise a self-sealing pierceable portion or element. The self-sealing pierceable portion or element may be integral with the closure 3 or may be at least partially removeable therefrom. The self-sealing pierceable portion or element may be formed from a resilient material, for example silicone.

Any of the above embodiments may be used to re-suspend (e.g. to re mix) particulate matter 60 with a liquid (e.g. a filtrate). In use, a sample of particulate matter 60 is placed in the container2, beneath the piston 41, and a liquid is poured into the container2. The piston 41 is then withdrawn through the liquid, such that the liquid passes though the filter 43 of the piston 41 and mixes with the particulate matter 60, thereby re-suspending that matter 60. The liquid (and/or a fluid or other material) may be introduced into the container 2 through the valve 37 or by at least partially removing the closure 3 from the container 2.

Additionally or alternatively, the liquid (and/or another fluid and/or other material) may be introduced into the container 2 through the aperture of an aperture and cooperating bung or cap (where provided). In use, the bung or cap is at least partially removed from the aperture and liquid, fluid and/or other material is introduced through the aperture into the container 2. Subsequent to introduction of the liquid, fluid and/or other material the bung or cap is inserted or reinserted in and/or on the aperture.

Additionally or alternatively, the liquid (and/or another fluid and/or other material) may be introduced into the container 2 through a self-sealing pierceable portion or element (where provided). In use, the self-sealing pierceable portion or element is pierced and/or penetrated by a piercing device such as a needle or syringe. Liquid, fluid and/or other material is then introduced into the container 2. Subsequent to introduction of the liquid, fluid and/or other material the piercing device is removed from the self-sealing pierceable portion or element which then reseals, e.g. in a fluid tight manner.

Additionally or alternatively, liquid, filtrate, fluid or other material may be removed from the container 2 by way of any of the above described means.

It will also be appreciated by those skilled in the art that any number of combinations of the aforementioned features and/or those shown in the appended drawings provide clear advantages over the prior art and are therefore within the scope of the invention described herein.

## Claims

1. A filtration device comprising a container with an open end and a closed end which is integral with the container, a lid for sealingly closing the open end of the container, a piston reciprocable within the container between the open end and the closed end and an actuating element connected to the piston and extending through a hole in the lid, the piston comprising a filter element bound by a peripheral portion with sealing means for sealingly engaging the container such that movement, in use, of the piston within the container forces a suspension fluid through the filter element but prevents the passage of biological particulates therethrough, wherein the device comprises a further sealing means between the lid and the actuating element, thereby to enable the device to be oriented with the lid of the container lowermost without allowing egress of suspension fluid.

2. Device according to claim 1, wherein the lid comprises a valve means through which filtrate may be introduced and/or removed.

3. Device according to claim 1 or 2, wherein at least a portion of the lid comprises a resilient material for sealingly engaging with the open end of the container.

4. Device according to claim 1, 2 or 3, wherein at least a portion of the lid comprises a resilient material for sealingly engaging with the actuating element.

5. Device according to any preceding claim, wherein the container comprises a formation about at least a portion of the open end that engages with a cooperating formation in or on the lid.

6. Device according to claim 5, wherein the formation of the container comprises an outwardly projecting radial bead and the lid comprises a top wall and a skirt depending therefrom, which skirt comprises a recess or lip for engaging the bead.

7. Device according to any preceding claim, wherein the lid comprises a formation adjacent to and/or extending inwardly of the hole for engaging with the actuating element, for example wherein the formation comprises a radial wiper seal.

8. Device according to any preceding claim, wherein the filter element is configured to prevent the passage therethrough of biological particulates larger than 0.5 nm or 1 nm or 5 nm or 0.1 µm or 0.2 µm or 9 µm or 100 µm or 1000 µm.

9. Device according to any preceding claim, wherein the mesh size of the filter element is between 0.5 nm and 1000 µm or between 0.1 µm and 100 µm or between 0.1 µm and 10 µm.

10. Device according to any preceding claim further comprising a stop adjacent the closed end against which the piston abuts to provide a predetermined volume between the piston and the closed end.

11. Device according to any preceding claim, wherein the closed end tapers to provide a predetermined volume into which movement of the piston is prevented.

12. Device according to any preceding claim, wherein the sealing means of the peripheral portion comprises one or more radial projections formed of a resilient material.

13. A method of filtering a biological sample, the method comprising inverting a sealed container having a lid sealed with respect to the container and with respect to an actuating element to prevent egress of suspension fluid and urging the actuating element through a hole in the lid of the sealed container to force a piston comprising a filter element bound by a peripheral sealing portion along a container such that a suspension fluid is urged through the filter element whilst the passage of biological particulates therethrough is prevented.

14. Method according to claim 14, wherein the container contains a liquid sample and air between the piston and a closed end of the container before the container is inverted, the step of inverting the container comprising orienting the container such that the liquid sample rests on the piston with the air located between the liquid sample and the closed end of the container.

15. Method according to claim 14 comprising urging the actuating element such that the air is compressed to create a positive pressure that forces liquid from the liquid sample through the filter element.

## Patentansprüche

1. Filtrierungsvorrichtung, umfassend einen Behälter mit einem offenen Ende und einem geschlossenen Ende, das einstückig mit dem Behälter ist, einen Deckel, zum abdichtenden Verschließen des offenen Endes des Behälters, einen im Behälter zwischen dem offenen Ende und dem geschlossenen Ende hin- und herbewegbaren Kolben und ein Betätigungselement, das mit dem Kolben verbunden ist und sich durch ein Loch im Deckel erstreckt, wobei der Kolben ein durch einen peripheren Abschnitt mit Dichtungsmitteln begrenztes Filterelement zum abdichtenden Ineingriffhehmen des Behälters umfasst, so dass eine Bewegung des Kolbens in Gebrauch innerhalb des Behälters ein Suspensionsfluid durch das Filterelement zwingt, aber das Passieren biologischer Partikel dadurch verhindert, wobei die Vorrichtung ein weiteres Dichtungsmittel zwischen dem Deckel und dem Betätigungselement umfasst, wodurch eine niedrigste Ausrichtung der Vorrichtung auf den Deckel des Behälters ermöglicht wird, ohne ein Austreten von Suspensionsfluid zuzulassen.

2. Vorrichtung nach Anspruch 1, wobei der Deckel ein Ventilmittel umfasst, durch das Filtrat eingeführt und/oder entfernt werden kann.

3. Vorrichtung nach Anspruch 1 oder 2, wobei mindestens ein Abschnitt des Deckels ein elastisches Material zum abdichtenden Ineingriffnehmen des offenen Endes des Behälters umfasst.

4. Vorrichtung nach Anspruch 1, 2 oder 3, wobei mindestens ein Abschnitt des Deckels ein elastisches Material zum dichtenden Ineingriffnehmen des Betätigungselements umfasst.

5. Vorrichtung nach einem der vorstehenden Ansprüche, wobei der Behälter eine Formierung um mindestens einen Abschnitt des offenen Endes umfasst, die eine kooperierende Formierung in oder auf dem Deckel in Eingriff nimmt.

6. Vorrichtung nach Anspruch 5, wobei die Formierung des Behälters einen nach außen vorstehenden radialen Wulst umfasst und der Deckel eine obere Wand und eine davon herabhängende Blende umfasst, wobei die Blende eine Aussparung oder eine Lippe umfasst, um den Wulst in Eingriff zu nehmen.

7. Vorrichtung nach einem der vorstehenden Ansprüche, wobei der Deckel eine Formierung umfasst, die an das Loch angrenzt und/oder sich im Inneren des Lochs erstreckt, um das Betätigungselement in Eingriff zu nehmen, beispielsweise wobei die Formierung eine radiale Abstreifdichtung umfasst.

8. Vorrichtung nach einem der vorstehenden Ansprüche, wobei das Filterelement konfiguriert ist, um das Passieren von biologischen Partikeln dadurch zu verhindern, die größer als 0,5 nm oder 1 nm oder 5 nm oder 0,1 µm oder 0,2 µm oder 9 µm oder 100 µm oder 1000 µm sind.

9. Vorrichtung nach einem der vorstehenden Ansprüche, wobei die Maschenweite des Filterelements zwischen 0,5 nm 1000 µm oder zwischen 0,1 µm und 100 µm oder zwischen 0,1 µm und 10 µm ist.

10. Vorrichtung nach einem der vorstehenden Ansprüche, ferner umfassend einen Anschlag angrenzend an das geschlossene Ende, gegen den der Kolben anstößt, um ein vorbestimmtes Volumen zwischen dem Kolben und dem geschlossenen Ende bereitzustellen.

11. Vorrichtung nach einem der vorstehenden Ansprüche, wobei sich das geschlossene Ende verjüngt, um ein vorbestimmtes Volumen bereitzustellen, in das eine Bewegung des Kolbens verhindert wird.

12. Vorrichtung nach einem der vorstehenden Ansprüche, wobei das Dichtungsmittel des peripheren Abschnitts einen oder mehrere radiale Vorsprünge umfasst, die aus einem elastischen Material geformt sind.

13. Verfahren zum Filtern einer biologischen Probe, wobei das Verfahren das Umkehren eines abgedichteten Behälters umfasst, der einen bezüglich des Behälters und bezüglich eines Betätigungselements abgedichteten Deckel aufweist, um ein Austreten von Suspensionsfluid zu verhindern und das Betätigungselement durch ein Loch im Deckel des abgedichteten Behälters zu drängen, um einen ein durch einen peripheren Dichtungsabschnitt entlang eines Behälters begrenztes Filterelement umfassenden Kolben zu zwingen, so dass ein Suspensionsfluid durch das Filterelement gedrängt wird, während das Passieren biologischer Partikel dadurch verhindert wird.

14. Verfahren nach Anspruch 14, wobei der Behälter vor dem Umkehren des Behälters eine flüssige Probe und Luft zwischen dem Kolben und einem geschlossenen Ende des Behälters enthält, wobei der Schritt des Umkehrens des Behälters das Ausrichten des Behälters, so dass die flüssige Probe auf dem Kolben ruht, während sich die Luft zwischen der flüssigen Probe und dem geschlossenen Ende des Behälters befindet, umfasst.

15. Verfahren nach Anspruch 14, umfassend das Drängen des Betätigungselements, so dass die Luft komprimiert wird, um einen positiven Druck zu erzeugen, der Flüssigkeit aus der flüssigen Probe durch das Filterelement zwingt.

## Revendications

1. Dispositif de filtration comprenant un récipient avec une extrémité ouverte et une extrémité fermée faisant partie intégrante du récipient, un couvercle assurant la fermeture étanche de l'extrémité ouverte du récipient, un piston à mouvement alternatif au sein du récipient, entre l'extrémité ouverte et l'extrémité fermée, et un élément actionneur raccordé au piston et déployé à travers un orifice du couvercle, le piston comprenant un élément filtrant tenu par une partie périphérique avec un élément d'étanchéité pour l'engagement étanche du récipient, de sorte qu'en cours d'usage le mouvement du piston au sein du récipient force un fluide en suspension à travers l'élément filtrant tout en empêchant le passage à travers de particules biologiques, le dispositif comprenant un autre élément d'étanchéité situé entre le couvercle et l'élément actionneur, en permettant ainsi l'orientation du dispositif avec le couvercle du récipient dans sa position inférieure, sans permettre la sortie de fluide en suspension.

2. Dispositif selon la revendication 1, le couvercle comprenant une vanne par laquelle du filtrat peut être introduit et/ou enlevé.

3. Dispositif selon la revendication 1 ou la revendication 2, une partie du couvercle comprenant une matière élastique pour l'engagement hermétique avec l'extrémité ouverte du récipient.

4. Dispositif selon la revendication 1, 2 ou 3, au moins une partie du couvercle comprenant une matière élastique pour l'engagement hermétique avec l'élément actionneur.

5. Dispositif selon une quelconque des revendications précédentes, le conteneur comprenant une formation autour d'au moins une partie de l'extrémité ouverte s'engageant avec une formation coopérant dans ou sur le couvercle.

6. Dispositif selon la revendication 5, la formation du conteneur comprenant un cordon radial saillant vers l'extérieur, et le couvercle comprenant une paroi supérieure et une bordure tributaire de celle-ci, ladite bordure comprenant un évidement ou un rebord pour l'engagement du cordon.

7. Dispositif selon une quelconque des revendications précédentes, le couvercle comprenant une formation adjacente au trou et/ou s'étendant vers l'intérieur de celui-ci pour l'engagement avec l'élément actionneur, par exemple lorsque la formation comprend un joint racleur radial.

8. Dispositif selon une quelconque des revendications précédentes, l'élément filtrant étant configuré pour empêcher le passage à travers celui-ci de particules biologiques mesurant plus de 0,5 nm ou 1 nm ou 5 nm ou 0,1 µm ou 0,2 µm ou 9 µm ou 100 µm ou 1000 µm.

9. Dispositif selon une quelconque des revendications précédentes, le maillage de l'élément filtrant étant compris entre 0,5 nm et 1000 µm ou entre 0,1 µm et 100 µm ou entre 0,1 µm et 10 µm.

10. Dispositif selon une quelconque des revendications précédentes, comprenant en outre une butée adjacente à l'extrémité fermée contre laquelle vient buter le piston pour fournir un volume prédéterminé entre le piston et l'extrémité fermée.

11. Dispositif selon une quelconque des revendications précédentes, l'extrémité fermée s'amincissant pour fournir un volume prédéterminé dans lequel le mouvement du piston est empêché.

12. Dispositif selon une quelconque des revendications précédentes, l'élément d'étanchéité de la partie périphérique comprenant une ou plusieurs saillies radiales réalisées avec un matériau élastique.

13. Méthode de filtrage d'un échantillon biologique, la méthode comprenant l'inversion d'un récipient scellé possédant un rebord scellé relativement au récipient, et relativement à un élément actionneur pour empêcher la sortie d'un fluide en suspension, et la sollicitation de l'élément actionneur à travers un orifice dans le couvercle du récipient scellé pour forcer un piston comprenant un élément filtrant tenu par une partie d'étanchéité périphérique le long d'un récipient, de façon à solliciter un fluide en suspension à travers l'élément filtrant tout en empêchant le passage à travers de particules biologiques.

14. Méthode selon la revendication 14, le récipient contenant un échantillon liquide et de l'air entre le piston et une extrémité fermée du récipient préalablement à l'inversion du récipient, l'étape d'inversion du récipient comprenant l'orientation du récipient de sorte que l'échantillon liquide soit posé sur le piston et l'air soit situé entre l'échantillon liquide et l'extrémité fermée du récipient.

15. Méthode selon la revendication 14, comprenant la sollicitation de l'élément actionneur de sorte que l'air soit comprimé afin de créer une pression positive forçant du liquide de l'échantillon liquide à travers l'élément filtrant.
